(19) **Europäisches Patentamt / European Patent Office / Office européen des brevets**

(11) **EP 4 450 103 B1**

(12) **FASCICULE DE BREVET EUROPEEN**

(45) Date de publication et mention
de la délivrance du brevet:
**04.03.2026 Bulletin 2026/10**

(21) Numéro de dépôt: **24165056.3**

(22) Date de dépôt: **21.03.2024**

(51) Classification Internationale des Brevets (IPC):
***A61M 16/00*** *(2006.01)*    ***A61M 16/20*** *(2006.01)*
***A61H 31/00*** *(2006.01)*

(52) Classification Coopérative des Brevets (CPC):
**A61M 16/024; A61M 16/0066; A61M 16/202;**
A61M 16/0833; A61M 16/125; A61M 16/205;
A61M 2016/0027; A61M 2016/0039;
A61M 2016/0042; A61M 2202/0208; A61M 2205/05;
A61M 2205/3331; A61M 2205/3365;
A61M 2205/505; A61M 2205/8206;    (Cont.)

(54) **VENTILATEUR MÉDICAL AVEC MODES VENTILATOIRES ADAPTÉS AU MASSAGE CARDIAQUE**

MEDIZINISCHES BEATMUNGSGERÄT MIT BEATMUNGSMODI GEEIGNET FÜR HERZMASSAGE

MEDICAL VENTILATOR WITH VENTILATORY MODES SUITABLE FOR CARDIAC MASSAGE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **19.04.2023 FR 2303930**

(43) Date de publication de la demande:
**23.10.2024 Bulletin 2024/43**

(73) Titulaire: **Air Liquide Medical Systems**
**92182 Antony Cedex (FR)**

(72) Inventeurs:
• **LESIMPLE, Arnaud**
**92182 Antony (FR)**
• **RIGOLLOT, Marceau**
**92182 Antony (FR)**
• **RICHARD, Jean-Christophe**
**92182 Antony (FR)**
• **BROC, Alexandre**
**92182 Antony (FR)**
• **LHUILLERY, Cyril**
**92182 Antony (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
EP-A1- 3 636 307    WO-A1-2016/174318
FR-A1- 3 087 127    US-A1- 2022 168 524
US-A1- 2022 347 425

   **(Cont. page suivante)**

(52) Classification Coopérative des Brevets (CPC):
(Cont.)A61M 2230/432

## Description

L'invention se rapporte à un ventilateur médical, c'est-à-dire un appareil de ventilation artificielle, équipé d'une micro-soufflante, afin d'apporter une assistance ventilatoire adaptée à une personne, i.e. un patient, en arrêt cardiaque lors d'une réanimation cardiopulmonaire (RCP).

L'arrêt cardiaque est une cause fréquente de mortalité chez l'homme. La prise en charge de l'arrêt cardiaque, appelée réanimation cardiopulmonaire (RCP), est basée sur une mise en œuvre d'un massage cardiaque et simultanément d'une ventilation assistée de la personne en arrêt cardiaque, aussi appelée patient.

Un massage cardiaque comprend une alternance de compressions et décompressions thoraciques opérées par un ou des secouristes, e.g. du personnel soignant. Les compressions thoraciques opérées durant le massage cardiaque, permettent de suppléer la pompe cardiaque pour faire circuler le sang dans les différents organes, en particulier l'irrigation du cerveau. Elles doivent donc être pratiquées le plus rapidement possible sur la personne en arrêt cardiaque. Une RCP de qualité permet d'augmenter les chances de survie du patient et est définie par des recommandations internationales. Ainsi, le massage doit être pratiqué à une fréquence d'environ 100 à 120 coups/minutes avec une profondeur de compression entre 4 et 6 cm environ.

Par ailleurs, une ventilation assistée doit également être délivrée au patient par le personnel soignant afin de favoriser les échanges gazeux pulmonaires avec le sang, c'est-à-dire un apport d'oxygène et une élimination du $CO_2$. La ventilation peut être dispensée au moyen d'un insufflateur de gaz manuel, c'est-à-dire un ballon auto-remplisseur à valve unidirectionnelle ou BAVU, ou d'un ventilateur médical, c'est-à-dire un appareil d'assistance respiratoire, servant à dispenser de l'air, de préférence enrichi en $O_2$, au patient, notamment pendant tout le massage cardiaque.

La délivrance de la ventilation, lors d'une RCP, est difficile du fait de l'application simultanée des compressions thoraciques pendant le massage cardiaque. Les ventilateurs médicaux conventionnels ne sont pas adaptés à cette situation et, si on les utilise durant une RCP, ils émettent des alarmes et/ou dysfonctionnent pendant les compressions thoraciques.

De plus, des réglages spécifiques de ventilation doivent être mis en œuvre afin de délivrer une ventilation efficace. En effet, il a été montré que, si elle est mal effectuée, la ventilation d'un patient en arrêt cardiaque peut être délétère pour la circulation générée par les compressions thoraciques et pour les échanges gazeux, en particulier si elle est délivrée de manière excessive ou, à l'inverse, insuffisante.

De même, lorsque le patient est réanimé du fait de la RCP, il est nécessaire de modifier, lors du retour d'activité cardiaque spontanée ou RACS, la manière de ventiler le patient afin de ne pas être délétère. En effet, les compressions thoraciques ne sont alors plus appliquées puisque le massage cardiaque cesse d'être pratiqué et le patient peut montrer des signes d'efforts respiratoires qui doivent être accompagnés. La fraction inspirée en oxygène ($FiO_2$) qui est délivrée au patient doit également être adaptée à cette situation spécifique, i.e. de RACS. Ainsi, en pratique, il est nécessaire de mettre une $FiO_2$ maximale (i.e. 100%) pendant l'arrêt cardiaque avec l'application des compressions thoraciques, mais en cas de RACS, il est nécessaire de diminuer la $FiO_2$ pour éviter une hyperoxie potentiellement délétère pour le patient.

Toutefois, un RACS peut n'être que temporaire. Ainsi, lors d'une RCP, un patient peut osciller entre des phases d'arrêt cardiaque et de RACS de manière récurrente, nécessitant à chaque fois une adaptation de la ventilation délivrée ainsi que des paramètres monitorés par le ventilateur.

Il est donc indispensable de pouvoir ventiler les patients en arrêt cardiaque de manière autonome, sûre et protectrice, donc de pouvoir adapter la ventilation du patient en fonction de la situation rencontrée, c'est-à-dire arrêt cardiaque ou RACS.

EP3636307 propose un ventilateur médical comprenant des moyens de sélection de la catégorie de patients et de modes ventilatoires compatibles, notamment un mode de réanimation cardio-pulmonaire (CPV). Dans ce mode, on peut choisir les niveaux ou valeurs de pression haute, de pression expiratoire (PEP), de $FiPO_2$, de fréquence respiratoire ou autre. Toutefois, ce document ne donne aucune indication sur la manière de choisir ces niveaux pour assurer une ventilation efficace en mode CPV.

Par ailleurs, EP3218037 et EP3323457 proposent un ventilateur médical, dans lequel le personnel soignant peut changer les réglages ventilatoires à utiliser en cas de massage cardiaque ou en l'absence/arrêt de massage cardiaque. Pour ce faire, on peut sélectionner des modes ventilatoires enregistrés dans le ventilateur qui sont basés sur des paramètres (e.g. pressions haute et basse, fréquence) applicables en cas de massage cardiaque ou, à l'inverse, en l'absence de massage cardiaque. Or, en pratique, les réglages proposés se sont montrés peu adaptés dans certains cas, notamment en phase de RACS, conduisant notamment à des activations d'alarmes non souhaitables.

La présente invention vise donc à proposer un ventilateur médical servant à ventiler artificiellement une personne, i.e. un patient, en arrêt cardiaque durant une RCP; qui soit amélioré, en particulier par rapport à ceux décrits par EP3218037 et EP3323457, de manière à améliorer la ventilation du patient tout en évitant ou minimisant le déclenchement d'alarmes intempestives.

Une solution selon l'invention concerne donc un ventilateur médical, c'est-à-dire un appareil d'assistance respiratoire ou ventilatoire, configuré pour fournir une assistance respiratoire à une personne en arrêt cardiaque, i.e. un patient, comprenant :

- une (micro-)soufflante motorisée configurée pour fournir un gaz respiratoire,
- un circuit de gaz interne comprenant une branche inspiratoire configurée pour acheminer le flux de gaz fourni par la soufflante motorisée,
- des moyens de mémorisation de modes ventilatoires (MV1, MV2) configurés pour mémoriser plusieurs modes ventilatoires comprenant au moins :
- un premier mode ventilatoire (MV1) à mettre en œuvre pendant une réalisation de massage cardiaque, se caractérisant par des valeurs de première pression basse (PB1) et de première pression haute (PH1), avec PH1>PB1 et
- un deuxième mode ventilatoire (MV2) à mettre en œuvre en l'absence de réalisation de massage cardiaque, se caractérisant par des valeurs de deuxième pression basse (PB2) et de deuxième pression haute (PH2), avec : PH2>PB2,
- des moyens de sélection de mode ventilatoire (MV1, MV2) permettant à un utilisateur de sélectionner au moins le premier (MV1) ou le deuxième (MV2) mode ventilatoire mémorisé, et
- des moyens de pilotage configurés pour piloter la soufflante motorisée en réponse à la sélection par l'utilisateur d'un desdits modes ventilatoire (MV1, MV2), pour fournir le gaz aux pressions basse et haute (PB1, PH1 ; PB2, PH2) correspondant au mode ventilatoire (MV1, MV2) sélectionné.

Selon l'invention, le deuxième mode ventilatoire (MV2) mémorisé se caractérise par une valeur de deuxième pression haute (PH2), telles que : PH2 < PH1.

En effet, en étudiant les problèmes rencontrés avec le type de ventilateur décrit par EP3218037 et EP3323457, les inventeurs de la présente invention se sont rendus compte que la configuration proposée n'était pas adaptée étant donné qu'en phase de RACS, c'est-à-dire après interruption du massage cardiaque, donc en l'absence de massage cardiaque, le patient peut générer lui-même de la ventilation spontanée, en plus de la ventilation délivrée par le ventilateur

Or, selon ces documents antérieurs, lors de la phase de RACS, c'est-à-dire en l'absence/après arrêt de massage cardiaque, il est préconisé de délivrer une seconde pression haute, appelée PH2, supérieure à la première pression haute, appelée PH1, mise en œuvre en phase d'arrêt cardiaque, c'est-à-dire pendant le massage cardiaque.

Le fait d'augmenter, après le massage cardiaque, cette seconde pression haute PH2 pose dès lors problème car cette seconde pression haute PH2 plus élevée que la pression PH1, mise en œuvre pendant le massage, peut venir se superposer à la ventilation spontanée générée par le patient lors du RACS, en générant potentiellement des activations d'alarmes intempestives au niveau du ventilateur.

A l'inverse, le fait de paramétrer dans le ventilateur, une valeur de deuxième pression haute (PH2), selon l'invention, inférieure à la première pression haute PH1 permet d'éviter ces problèmes. De plus, si l'application des compressions thoraciques en phase d'arrêt cardiaque, c'est-à-dire pendant le massage cardiaque, a tendance à réduire les volumes délivrés et donc justifie l'application d'une pression haute PH1, après arrêt du massage, cette réduction des volumes n'existe plus et il est alors souhaitable de baisser la pression appliquée, i.e. PH2.

Selon le mode de réalisation considéré, le ventilateur de l'invention peut comprendre l'une ou plusieurs des caractéristiques suivantes :

- la première pression basse (PB1) est comprise entre 0 et 10 cmH$_2$O, de préférence entre 0 et 5 cmH$_2$O.
- la première pression haute (PH1) est comprise entre 10 et 40 cmH$_2$O, de préférence entre 20 et 30 cmH$_2$O.
- la seconde pression basse (PB2) est comprise entre 0 et 20 cmH$_2$O, de préférence entre 5 et 10 cmH$_2$O.
- la seconde pression haute (PH2) est comprise entre 10 et 40 cmH$_2$O, de préférence entre 15 et 25 cmH$_2$O.
- le premier mode ventilatoire (MV1) se caractérise en outre par une première fréquence (F1) et le second mode ventilatoire (MV2) se caractérise en outre par une deuxième fréquence (F2) données, avec F2>F1.
- la première fréquence (F1) est comprise entre 5 et 20 cycles/min, de préférence entre 8 et 12 cycles/min.
- la deuxième fréquence (F2) est comprise entre 5 et 35 cycles/min, de préférence entre 15 et 20 cycles/min.
- il comprend un écran d'affichage à dalle digitale affichant au moins une touche virtuelle.
- les moyens de sélection de mode ventilatoire comprennent ladite au moins une touche virtuelle.
- l'écran d'affichage est à affichage en couleurs ou en noir et blanc, de préférence en couleurs.
- il comprend en outre des moyens de mesure de pression agencés dans le circuit de gaz pour y opérer une ou des mesures de pression.
- les moyens de mesure de pression coopèrent avec les moyens de pilotage pour leur fournir la ou les mesures de pression.
- les moyens de mesure de pression sont reliés électriquement aux moyens de pilotage.
- les moyens de mesure de pression comprennent un (des) capteur de pression.
- les moyens de pilotage traitent la ou les mesures de pression provenant du (des) capteur de pression pour déterminer la pression dans le circuit de gaz.
- les moyens de pilotage traitent la ou les mesures de pression pour délivrer la pression réglée , i.e. PB1, PB2, PH1 et PH2.
- les moyens de pilotage comprennent au moins un microprocesseur.
- ledit au moins un microprocesseur est agencé sur une carte électronique.

- les moyens de mémorisation de modes ventilatoires (MV1, MV2) comprennent au moins une mémoire informatique, en particulier une mémoire flash ou analogue.
- la soufflante comprend un moteur électrique.
- le circuit de gaz comprend une branche inspiratoire et une branche expiratoire.
- les branches inspiratoire et expiratoire sont reliées l'une à l'autre au niveau d'une pièce de jonction, telle une pièce en Y.
- le circuit de gaz, en particulier la branche inspiratoire et la branche expiratoire, comprend un ou des passages de gaz, telles un ou des conduits de gaz, notamment des tuyaux flexibles ou analogues.
- la branche expiratoire est configurée pour recueillir le gaz expiré par le patient (i.e. gaz riche en $CO_2$) et l'évacuer vers l'atmosphère via un orifice de sortie de gaz.
- la branche expiratoire comprend une vanne expiratoire, de préférence une électrovanne, commandée par les moyens de pilotage.
- il comprend un dispositif de mesure de teneur en $CO_2$ pour mesurer la teneur (i.e. quantité) en $CO_2$ dans le gaz sortant des poumons du patient, en particulier un capnomètre.
- le dispositif de mesure de teneur en $CO_2$ coopère avec les moyens de pilotage pour leur fournir des mesures de teneur en $CO_2$.
- les moyens de pilotage sont configurés pour traiter les mesures de teneur en $CO_2$ et commander un affichage sur l'écran d'affichage, d'une courbe de teneur en $CO_2$ au fil du temps.
- le dispositif de mesure de teneur en $CO_2$ est agencé sur la branche expiratoire.
- le dispositif de mesure de teneur en $CO_2$ est relié électriquement aux moyens de pilotage.
- il comprend des moyens d'alimentation électrique, notamment une batterie rechargeable ou un raccordement au secteur (110/220V), alimentant notamment les moyens de pilotage, le moteur électrique de la soufflante et l'écran d'affichage.
- le ventilateur comprend un boitier externe formant carcasse rigide.
- selon le cas, les première et seconde pressions basses PB, PB2 peuvent être telles que : PB1=PB2, PB1<PB2 ou PB1>PB2.
- avantageusement, les première et seconde pressions basses (PB, PB2) sont telles que : PB1=PB2.
- le dispositif de mesure de teneur en $CO_2$ est un capnomètre.
- le deuxième mode ventilatoire (MV2) se caractérise par des valeurs de deuxième pression basse (PB2) et de deuxième pression haute (PH2) correspondant à une ventilation à mettre en œuvre après détection d'un RACS du patient.
- l'écran d'affichage est configuré pour afficher la courbe de teneur en $CO_2$ au fil du temps et la sélection du deuxième mode ventilatoire (MV2) par l'utilisateur est opérée, via les moyens de sélection, après visualisation (i.e. après apparition) d'un RACS du patient sur la courbe de teneur en $CO_2$ au fil du temps affichée.
- un RACS du patient se caractérise sur la courbe de teneur en $CO_2$ au fil du temps par une augmentation notable de la quantité de $CO_2$ sortant des poumons du patient P.
- la sélection du deuxième mode ventilatoire (MV2) par l'utilisateur est opérée par appui digital (i.e. avec un doigt, typiquement son index) sur la touche virtuelle affichée (i.e. touche tactile) sur l'écran d'affichage à dalle tactile.

L'invention va maintenant être mieux comprise grâce à la description détaillée suivante, faite à titre illustratif mais non limitatif, en référence aux figures annexées parmi lesquelles :

Fig. 1 schématise les niveaux de pression mis en œuvre par le ventilateur médical de Fig. 2 en présence ou en l'absence de massage cardiaque.
Fig. 2 schématise un ventilateur médical selon l'invention.
Fig. 3 schématise une courbe de $CO_2$ au fil du temps (en min) montrant un RACS.

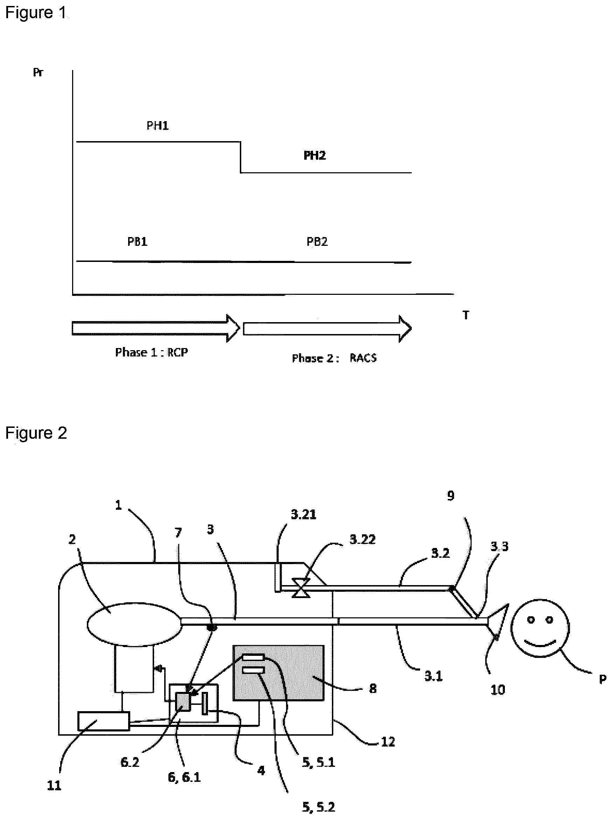

Fig. 2 schématise un mode de réalisation d'un appareil d'assistance ventilatoire ou ventilateur médical 1 selon l'invention, permettant d'apporter une assistance ventilatoire adaptée à une personne, i.e. un patient P, en arrêt cardiaque, lors de la mise en œuvre d'une réanimation cardiopulmonaire (RCP) pratiquée par un ou des secouristes, typiquement des personnels soignants de type médecin, infirmière ou analogue.

Il permet d'apporter une ventilation mécanique adaptée pendant ou après la réalisation d'un massage cardiaque, c'est-à-dire de couvrir toutes les étapes de la RCP, incluant la ou les phases de massage cardiaque comprenant les compressions et les relâchements thoraciques, et la ou les phases d'absence ou d'arrêt de massage, en particulier lors d'un retour d'activité cardiaque spontanée (RACS) chez le patient P, pour améliorer la ventilation en évitant les interruptions de ventilation dues à des alarmes intempestives et/ou toute ventilation inadaptée, notamment excessive durant la ou les phases d'arrêt de massage cardiaque, notamment lors d'un RACS.

Le ventilateur médical 1 comprend une (micro-) soufflante motorisée 2, aussi appelée turbine ou compresseur, en tant que source de gaz délivrant un flux de gaz d'assistance respiratoire, c'est-à-dire un gaz respiratoire, typiquement un flux d'air ou d'air enrichi en oxygène.

La soufflante motorisée 2 fournit le gaz respiratoire à un circuit de gaz 3, tel un (ou des) passage ou conduit de gaz interne, comprenant une branche inspiratoire 3.1 configurée pour acheminer le flux de gaz fourni par la soufflante motorisée 2 jusqu'au patient P. Autrement dit, le circuit de gaz 3 permet de relier fluidiquement la soufflante motorisée 2 aux voies aériennes du patient P, par l'intermédiaire d'une interface respiratoire 10, par exemple un masque respiratoire, une sonde d'intubation trachéale ou autre.

Classiquement, une soufflante motorisée 2 comprend un moteur électrique agencé dans un carter protecteur et piloté par les moyens de pilotage 5 du ventilateur 1, lequel moteur permet de mettre un axe-moteur en rotation, lequel porte une roue à ailettes servant à aspirer le gaz et à le fournir au circuit de gaz 3. La roue à ailettes est habituellement agencée dans le compartiment interne d'une volute surmontant le carter protecteur du moteur. Typiquement, le moteur peut être du type sans balai. Les vitesses de rotation du moteur peuvent être comprises entre 500 et 35 000 tr/min.

Le circuit de gaz 3 comprend également ici une branche expiratoire 3.2 conçue pour recueillir les gaz expirés par le patient P qui sont riches en $CO_2$ et les évacuer vers l'atmosphère via un orifice de sortie de gaz 3.21. La branche expiratoire 3.1 peut comprendre un capteur de débit expiratoire (non montré), par exemple un capteur à fil chaud, relié électriquement aux moyens de pilotage 6, ainsi qu'une valve expiratoire 3.22, telle une électrovanne, commandée par les moyens de pilotage 6. Les branches inspiratoire 3.1 et expiratoire 3.2 viennent se raccorder fluidiquement l'une à l'autre au niveau d'un élément de jonction 3.3, telle une pièce en Y, située en amont de l'interface respiratoire 10.

Le circuit de gaz 3 comprend aussi des moyens de mesure permettant de mesurer au moins un paramètre représentatif du flux de gaz choisi parmi la pression du gaz, le débit de gaz insufflé, le débit de gaz expiré par le patient P et la vitesse de rotation de la micro-soufflante, et à délivrer au moins un signal (ou valeur) représentatif dudit au moins un paramètre mesuré aux moyens de pilotage 6 afin d'y être utilisé notamment pour piloter la soufflante 2 ou autre. De préférence, au moins un (ou des) capteur de pression 7 est agencé pour mesurer la pression du gaz dans le circuit de gaz 3, typiquement dans la branche inspiratoire 3.1. Les mesures (signal ou valeur) sont transmises aux moyens de pilotage 6 qui les traitent.

Par ailleurs, est aussi prévu un dispositif de mesure de teneur en $CO_2$ 9, en particulier un capnomètre ou analogue, permettant de mesurer la teneur en $CO_2$ dans le gaz sortant des poumons du patient afin de pouvoir notamment détecter un RACS.

La transmission du (ou des) signal (ou valeurs) provenant des moyens de mesure et du dispositif de mesure de teneur en $CO_2$ 9, i.e. du capnomètre, aux moyens de pilotage 6 se fait via une connectique adaptée, c'est-à-dire des liaisons électriques, tel que câbles ou autres.

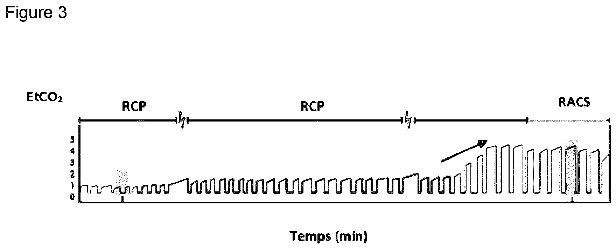

Les moyens de pilotage 6 traitent les mesures de teneur en $CO_2$ et commandent ensuite un affichage sur l'écran d'affichage 8, d'une courbe de teneur en $CO_2$ au fil du temps, c'est-à-dire des variations de la concentration en $CO_2$ présente dans les flux sortant des poumons du patient P, ce qui permet au personnel soignant de déterminer un RACS, qui se caractérise par une augmentation notable de la quantité de $CO_2$ sortant des poumons du patient P, comme illustré sur la Figure 3, laquelle schématise la quantité de $CO_2$ mesurée (Et-$CO_2$) provenant des poumons du patient au fil du temps (minutes), c'est-à-dire pendant la RCP puis au moment de la reprise d'activité cardiaque spontanée ou RACS.

Par ailleurs, les moyens de de pilotage 6 du ventilateur 1 peuvent notamment déduire ou déterminer à partir de tout ou partie des signaux transmis différentes informations, notamment un massage cardiaque en cours de réalisation, un volume de gaz insufflé au patient P, un volume de gaz expiré par le patient P.

Les moyens de pilotage 6 du ventilateur 1 comprennent une ou plusieurs cartes électroniques 6.1 portant ici un (des) microprocesseur 6.2 programmé notamment avec un (ou plusieurs) algorithme(s) de traitement pour traiter tout ou partie des différents signaux reçus, notamment opérer des calculs, des comparaisons, établir des courbes de suivi ou autre, et/ou agir en réponse à tout ou partie de ces signaux ou à leur traitement.

Sont prévus aussi des moyens de mémorisation 4, telle une (des) mémoire flash ou analogue, servant à enregistrer notamment des modes ventilatoires MV1, MV2.

En effet, étant donné que les niveaux de pression haute, de pression basse et de fréquence ventilatoire notamment, délivrés lors d'une phase de massage cardiaque sont différents de ceux délivrés lors d'une phase de non-massage cardiaque, par exemple suite à une interruption de massage du fait d'un RACS du patient, il est nécessaire de mémoriser différents modes ventilatoires MV1, MV2, lesquels sont utilisés par les moyens de pilotage 6 pour piloter le ventilateur 1, notamment pour commander la soufflante 2 délivrant le gaz d'assistance respiratoire.

Le basculement d'un premier mode ventilatoire MV1 à un second mode ventilatoire MV2 est opéré par actionnement de la part d'un utilisateur, tel un secouriste, i.e. personnel soignant, de moyens de sélection 5 de mode ventilatoire agencés sur le ventilateur 1 permettant de sélectionner le premier (MV1) ou le deuxième (MV2) mode ventilatoire qui sont mémorisés dans des moyens de mémorisation 4.

Pour ce faire, est prévu ici une interface homme-machine ou IHM comprenant un écran d'affichage 8, de préférence à dalle tactile, c'est-à-dire un écran digital, permettant d'afficher des informations utiles relatives à la ventilation délivrée, en particulier les réglages ventilatoires, par exemple les niveaux de pression haute et basse, la fréquence respiratoire, la fraction inspirée en oxygène, le temps d'insufflation, mais aussi les monitorages de différents paramètres ventilatoires, par exemple le volume insufflé, le volume expiré, la valeur de teneur en $CO_2$, la valeur de pression maximum atteinte lors de l'insufflation, le volume minute, et également des courbes de différents signaux ventilatoires, par exemple une courbe de teneur en $CO_2$ du gaz expiré, une courbe de débit des voies aériennes, une courbe de pression des voies aériennes, une courbe de volume ...

L'IHM comprend en outre les moyens de sélection 5, par exemple des boutons poussoirs ou rotatifs, des curseurs, des touches d'activation ou de sélection, ou analogue permettant à l'utilisateur d'opérer des choix, des réglages, des sélections, des confirmations ou autres.

Les moyens de sélection 5 permettent également de modifier, si besoin est, les paramètres de la ventilation mécanique proposés automatiquement par le ventilateur 1, voire de pouvoir indiquer au ventilateur 1, un changement de la nature du gaz mis en œuvre, par exemple le passage d'air à un mélange air/oxygène ou un changement de teneur en oxygène d'un mélange air/oxygène.

Avantageusement, les moyens de sélection 5 comprennent une ou plusieurs touches virtuelles 5.1, 5.2 s'affichant sur l'écran d'affichage 8 à dalle tactile de sorte que l'utilisateur puisse sélectionner le premier (MV1) ou le deuxième (MV2) mode ventilatoire désiré en appuyant sur une touche virtuelle 5.1, 5.2.

Le contact du doigt de l'utilisateur sur la (les) touche virtuelle 5.1, 5.2 affichée(s) sur l'écran d'affichage 8 à dalle tactile est reconnu par le ventilateur 1 et un signal correspondant est transmis aux moyens de pilotage 6 du ventilateur 1, typiquement au microprocesseur 6.2 porté par la carte électronique 6.1. Ceux-ci peuvent alors aller retrouver les paramètres du premier (MV1) ou du deuxième (MV2) mode ventilatoire sélectionné au sein des moyens de mémorisation 4, en particulier les couples de valeurs de pression haute et basse (PB1, PH1 ; PB2, PH2), et les utiliser pour piloter notamment la soufflante 2 de sorte qu'elle délivre le gaz respiratoire selon les niveaux de pression haute et basse désirés.

Par exemple, comme illustré en Fig. 1, représentant les pressions (Pr) mises en œuvre au fil du temps (T), au début d'une RCP avec massage cardiaque et ventilation assistée d'un patient en arrêt cardiaque, l'utilisateur va appuyer sur la touche virtuelle 5.1 s'affichant sur l'écran d'affichage 8 à dalle tactile du ventilateur 1 pour signaler aux moyens de pilotage 6 du ventilateur 1 qu'une RCP a débuté, donc qu'il doit lancer un premier mode ventilatoire (MV1) se caractérisant par des premières pressions basse PB1 et haute PH1 correspondant à des pressions à mettre en œuvre pendant la phase de massage cardiaque (Phase 1 : RCP), par exemple une première pression basse PB1 de 5 cmH2O et une première pression haute PH1 de 20 cmH2O.

La ventilation du patient se fait alors entre ces deux niveaux de pression pendant toute la phase de massage cardiaque (Phase 1) durant laquelle le patient est soumis à une alternance de compressions thoraciques et de relâchements (non détaillé sur Fig. 1).

Ensuite, après par exemple plusieurs minutes de massage cardiaque (Phase 1), on peut assister à un RACS du patient, comme illustré en Fig. 3. A ce moment, l'utilisateur, i.e. personnel soignant, va appuyer sur la touche virtuelle 5.2 s'affichant sur l'écran d'affichage 8 à dalle tactile pour signaler aux moyens de pilotage 6 du ventilateur 1 que la RCP a stoppé, c'est-à-dire qu'aucun massage cardiaque n'est (plus) en cours, et donc qu'il doit lancer un second mode ventilatoire (MV2) se caractérisant par des secondes pressions basse PB2 et haute PH2 correspondant à des pressions à mettre en œuvre en l'absence de massage cardiaque (Phase 2), par exemple une seconde pression basse PB2 de 5cmH2O et une seconde pression haute PH2 de 15 cmH2O. Ici les pressions basses PB1, PB2 sont égales, c'est-à-dire PB2 = PB1 ; toutefois, elles pourraient être différentes.

Autrement dit, la sélection du deuxième mode ventilatoire MV2 par l'utilisateur est opérée via les moyens de sélection 5 ; 5.1, 5.2, après visualisation du RACS du patient apparaissant sur la courbe de teneur en $CO_2$ affichée, i.e. une augmentation notable de la quantité de $CO_2$ sortant des poumons du patient P, comme visible sur la Fig. 3 (i.e. flèche).

Selon l'invention, il est primordial que PH2 soit inférieure à PH1. En effet, il a été mis en évidence, dans le cadre de la présente invention, qu'en phase de RACS, c'est-à-dire après interruption du massage cardiaque, i.e. en l'absence de massage cardiaque, le patient peut générer lui-même de la ventilation spontanée et qu'il convient alors de moduler la ventilation délivrée par le ventilateur 1 pour éviter des problèmes d'activation d'alarmes intempestives. De plus, en phase d'arrêt cardiaque, le massage cardiaque a tendance à réduire les volumes délivrés par l'application d'une pression haute PH1. Après arrêt du massage, cette réduction des volumes n'existe plus et il est alors souhaitable de baisser la pression appliquée, i.e. PH2, tel que PH2 < PH1.

Par ailleurs, lors du passage du premier mode ventilatoire (MV1) au second mode ventilatoire (MV2), la pression basse peut être, selon le cas, maintenue constante, i.e PB1=PB2 ; ou augmentée, i.e. PB2 > PB1, ou encore diminuée, i.e. PB2 < PB1.

En outre, la fréquence de ventilation, c'est-à-dire l'alternance des pressions haute et basse mise en œuvre peut aussi varier lors du passage du premier mode ventilatoire (MV1) au second mode ventilatoire (MV2), par exemple être augmentée en cas d'arrêt de massage cardiaque de manière à compenser la perte de ventilation provoquée par l'arrêt des compressions thoraciques du massage cardiaque, par exemple la fréquence de ventilation peut augmenter d'une fréquence initiale F1 de l'ordre de 10 cycles/min à une fréquence supérieure F2 de l'ordre de 15 cycles/min. A l'inverse, la fréquence peut repasser de F2 à F1 en cas de redémarrage des compressions thoraciques en cas de nouvel arrêt cardiaque.

Dit autrement, la fréquence F2 mise en œuvre en l'absence/arrêt de massage cardiaque (mode MW2), typiquement lors d'un RACS, et la F1 mise en œuvre pendant un massage cardiaque (mode MW1) sont telles que : F2 > F1.

Typiquement, F1 est comprise entre 8 et 12 cycles/min et F2 est comprise entre 15 et 20 cycles/min.

Par analogie, on peut également diminuer la fraction inspirée d'oxygène ($FiO_2$) en cas de détection d'arrêt du massage cardiaque, typiquement lors d'un RACS, par exemple la $FiO_2$ délivrée peut être de 50%. A l'inverse la $FiO_2$ peut être augmentée en cas de reprise des compressions thoraciques, par exemple en cas de nouvel arrêt cardiaque après un RACS, et peut alors repasser de 50% à 100%.

D'une façon générale, les éléments du ventilateur 1, notamment la soufflante 2, les moyens de pilotage 5, au moins une partie du circuit de gaz 3, l'écran d'affichage 8, sont agencés dans un boitier 12 formant une carcasse rigide du ventilateur médical 1.

Le ventilateur 1 et ses composants requérant de l'énergie pour fonctionner, notamment le moteur électrique de la soufflante 2, les moyens de pilotage 5, l'écran d'affichage 8, le ou les capteurs 7, 9, sont alimentés, directement ou indirectement, en courant électrique (110/220 V) issu d'une source de courant électrique 11, c'est-à-dire des moyens d'alimentation électrique, par exemple une ou plusieurs batteries rechargeables, une l'alimentation électrique d'un véhicule de secours ou le secteur électrique. Si besoin est, le ventilateur 1 peut intégrer aussi un convertisseur de courant conçu pour abaisser la tension d'alimentation.

Par ailleurs, le ventilateur médical 1 de l'invention peut aussi un dispositif mélangeur de gaz coopérant avec des capteurs de débit de manière à opérer un mélange un débit d'oxygène pur donné avec un débit d'air donné afin de délivrer une fraction inspirée en oxygène réglée de sorte d'adapter la fraction inspirée en oxygène délivrée au patient.

D'une façon générale, le ventilateur médical 1 de l'invention est particulièrement bien adapté à une utilisation dans le cadre d'une réanimation cardiopulmonaire (RCP) comprenant la mise en œuvre d'un massage cardiaque et simultanément d'une ventilation assistée de la personne en arrêt cardiaque, au moyen du ventilateur médical 1 de l'invention.

**Revendications**

1. Ventilateur médical (1) configuré pour fournir une assistance respiratoire à une personne en arrêt cardiaque comprenant :

   - une soufflante motorisée (2) configurée pour fournir un gaz respiratoire,
   - un circuit de gaz (3) comprenant une branche inspiratoire (3.1) configurée pour acheminer le flux de gaz fourni par la soufflante motorisée (2),

   - des moyens de mémorisation (4) de modes ventilatoires (MV1, MV2) configurés pour mémoriser plusieurs modes ventilatoires comprenant au moins :
   - un premier mode ventilatoire (MV1) à mettre en œuvre pendant une réalisation de massage cardiaque, **se caractérisant par** des valeurs de première pression basse (PB1) et de première pression haute (PH1), avec PH1>PB1 et
   - un deuxième mode ventilatoire (MV2) à mettre en œuvre en l'absence de réalisation de massage cardiaque, **se caractérisant par** des valeurs de deuxième pression basse (PB2) et de deuxième pression haute (PH2), avec PH2>PB2,
   - des moyens de sélection (5 ; 5.1, 5.2) de mode ventilatoire (MV1, MV2) permettant à un utilisateur de sélectionner au moins le premier (MV1) ou le deuxième (MV2) mode ventilatoire mémorisé, et
   - des moyens de pilotage (6) configurés pour piloter la soufflante motorisée (2) en réponse à la sélection par l'utilisateur d'un desdits modes ventilatoire (MV1, MV2), pour fournir le gaz aux pressions basse et haute (PB1, PH1 ; PB2, PH2) correspondant au mode ventilatoire (MV1, MV2) sélectionné,

   **caractérisé en ce que** le deuxième mode ventilatoire (MV2) mémorisé se **caractérise par** une valeur de deuxième pression haute (PH2), telles que :

   $$PH2 < PH1$$

2. Ventilateur selon la revendication 1, **caractérisé en ce que** :

   - la première pression basse (PB1) est comprise entre 0 et 10 $cmH_2O$, de préférence entre 0 et 5 $cmH_2O$ ;
   - la première pression haute (PH1) est comprise entre 10 et 40 $cmH_2O$, de préférence entre 20 et 30 $cmH_2O$ ;
   - la seconde pression basse (PB2) est comprise entre 0 et 20 $cmH_2O$, de préférence entre 5 et 10 $cmH_2O$ ; et/ou
   - la seconde pression haute (PH2) est comprise entre 10 et 40 $cmH_2O$, de préférence entre 15 et 25 $cmH_2O$.

3. Ventilateur selon la revendication 1, **caractérisé en ce que** le premier mode ventilatoire (MV1) se **caractérise en outre par** une première fréquence (F1) et le second mode ventilatoire (MV2) se **caractérise en outre par** une deuxième fréquence (F2) données, avec F2>F1.

**4.** Ventilateur selon la revendication 1, **caractérisé en ce que** :

- la première fréquence (F1) est comprise entre 5 et 20 cycles/min et/ou
- la deuxième fréquence (F2) est comprise entre 5 et 35 cycles/min.

**5.** Ventilateur selon la revendication 1, **caractérisé en ce qu'**il comprend un écran d'affichage (8) à dalle digitale affichant au moins une touche virtuelle (5.1, 5.2), les moyens de sélection (5 ; 5.1, 5.2) de mode ventilatoire comprenant ladite au moins une touche virtuelle (5.1, 5.2).

**6.** Ventilateur selon la revendication 1, **caractérisé en ce que** les moyens de mémorisation (4) de modes ventilatoires (MV1, MV2) comprennent au moins une mémoire informatique.

**7.** Ventilateur selon la revendication 1, **caractérisé en ce qu'**il comprend en outre des moyens de mesure de pression (7) agencés dans le circuit de gaz (3) pour y opérer une ou des mesures de pression et coopérant avec les moyens de pilotage (6) pour leur fournir la ou les mesures de pression.

**8.** Ventilateur selon la revendication 1, **caractérisé en ce que** les moyens de pilotage (6) comprennent au moins un microprocesseur (6.2), de préférence ledit au moins un microprocesseur (6.2) est agencé sur une carte électronique (6.1).

**9.** Ventilateur selon la revendication 1, **caractérisé en ce qu'**il comprend un dispositif de mesure de teneur en $CO_2$ (9) pour mesurer la teneur en $CO_2$ dans le gaz sortant des poumons du patient et transmettre les mesures de teneur en $CO_2$ aux moyens de pilotage (6), et les moyens de pilotage sont configurés pour traiter lesdites mesures de teneur en $CO_2$ et commander un affichage sur l'écran d'affichage (8), d'une courbe de teneur en $CO_2$ au fil du temps.

**10.** Ventilateur selon la revendication 1, **caractérisé en ce que** le circuit de gaz (3) est relié fluidiquement à une interface respiratoire (10), de préférence un masque respiratoire ou une sonde d'intubation trachéale.

**11.** Ventilateur selon l'une des revendications 1 ou 2, **caractérisé en ce que** les première et seconde pressions basses (PB, PB2) sont telles que : PB1=PB2.

**12.** Ventilateur selon la revendication 9, **caractérisé en ce que** le dispositif de mesure de teneur en $CO_2$ (9) est un capnomètre.

**13.** Ventilateur selon l'une des revendications 1 ou 4, **caractérisé en ce que** :

- la première fréquence (F1) est comprise entre 8 et 12 cycles/min et/ou
- la deuxième fréquence (F2) est comprise entre 15 et 20 cycles/min.

**14.** Ventilateur selon l'une des revendications 1 ou 2, **caractérisé en ce que** le deuxième mode ventilatoire (MV2) se **caractérise par** des valeurs de deuxième pression basse (PB2) et de deuxième pression haute (PH2) correspondant à une ventilation à mettre en œuvre après détection d'un RACS du patient.

**15.** Ventilateur selon les revendications 1, 9 et 14, **caractérisé en ce que** :

- l'écran d'affichage (8) est configuré pour afficher la courbe de teneur en $CO_2$ au fil du temps et
- la sélection du deuxième mode ventilatoire (MV2) par l'utilisateur est opérée via les moyens de sélection (5 ; 5.1, 5.2), après visualisation d'un RACS du patient sur la courbe de teneur en $CO_2$ au fil du temps affichée **se caractérisant par** une augmentation notable de la quantité de $CO_2$ sortant des poumons du patient P.

**Patentansprüche**

**1.** Medizinisches Beatmungsgerät (1), das zur Atemunterstützung einer Person mit Herzstillstand konfiguriert ist, mit:

- ein motorbetriebenes Gebläse (2), das zur Bereitstellung von Atemgas ausgelegt ist,
- einen Gaskreislauf (3) mit einem Einatemzweig (3.1), der so konfiguriert ist, dass er den vom motorbetriebenen Gebläse (2) gelieferten Gasstrom weiterleitet,
- Speichermittel (4) für Beatmungsmodi (MV1, MV2), die so konfiguriert sind, dass sie mehrere Beatmungsmodi speichern, darunter mindestens:
- einen ersten Beatmungsmodus (MV1), der während einer Herzdruckmassage anzuwenden ist und durch Werte für einen ersten niedrigen Druck (PB1) und einen ersten hohen Druck (PH1) gekennzeichnet ist, wobei PH1>PB1 ist, und
- einen zweiten Beatmungsmodus (MV2), der ohne Durchführung einer Herzdruckmassage anzuwenden ist und durch Werte für einen zweiten niedrigen Druck (PB2) und einen zweiten hohen Druck (PH2) gekennzeichnet ist, wobei PH2>PB2 ist,

- Auswahlmittel (5; 5.1, 5.2) für den Beatmungsmodus (MV1, MV2), die es einem Benutzer ermöglichen, mindestens den ersten (MV1) oder den zweiten (MV2) gespeicherten Beatmungsmodus auszuwählen, und

- Steuermittel (6), die so konfiguriert sind, dass sie das motorisierte Gebläse (2) in Reaktion auf die Auswahl eines der Beatmungsmodi (MV1, MV2) durch den Benutzer steuern, um das Gas mit den niedrigen und hohen Drücken (PB1, PH1; PB2, PH2) zu liefern, die dem ausgewählten Beatmungsmodus (MV1, MV2) entsprechen,

**dadurch gekennzeichnet, dass** der zweite gespeicherte Beatmungsmodus (MV2) durch einen zweiten hohen Druckwert (PH2) gekennzeichnet ist, sodass:

$$PH2 < PH1$$

2. Beatmungsgerät nach Anspruch 1, **dadurch gekennzeichnet, dass**:

- der erste niedrige Druck (PB1) zwischen 0 und 10 cmH$_2$O, vorzugsweise zwischen 0 und 5 cmH$_2$O liegt;
- der erste hohe Druck (PH1) zwischen 10 und 40 cmH$_2$O, vorzugsweise zwischen 20 und 30 cmH$_2$O liegt;
- der zweite niedrige Druck (PB2) zwischen 0 und 20 cmH$_2$O, vorzugsweise zwischen 5 und 10 cmH$_2$O liegt; und/oder
- der zweite hohe Druck (PH2) zwischen 10 und 40 cmH$_2$O, vorzugsweise zwischen 15 und 25 cmH$_2$O liegt.

3. Beatmungsgerät nach Anspruch 1, **dadurch gekennzeichnet, dass** der erste Beatmungsmodus (MV1) ferner durch eine erste Frequenz (F1) und der zweite Beatmungsmodus (MV2) ferner durch eine zweite Frequenz (F2) gekennzeichnet ist, wobei F2>F1 ist.

4. Beatmungsgerät nach Anspruch 1, **dadurch gekennzeichnet, dass**:

- die erste Frequenz (F1) zwischen 5 und 20 Zyklen/min liegt und/oder
- die zweite Frequenz (F2) zwischen 5 und 35 Zyklen/min liegt.

5. Beatmungsgerät nach Anspruch 1, **dadurch gekennzeichnet, dass** es einen Bildschirm (8) mit digitalem Display umfasst, der mindestens eine virtuelle Taste (5.1, 5.2) anzeigt, wobei die Auswahlmittel (5; 5.1, 5.2) für den Beatmungsmodus die mindestens eine virtuelle Taste (5.1, 5.2) umfassen.

6. Beatmungsgerät nach Anspruch 1, **dadurch gekennzeichnet, dass** die Speichermittel (4) für Beatmungsmodi (MV1, MV2) mindestens einen Computerspeicher umfassen.

7. Beatmungsgerät nach Anspruch 1, **dadurch gekennzeichnet, dass** es außerdem Druckmessmittel (7) umfasst, die im Gaskreislauf (3) angeordnet sind, um dort eine oder mehrere Druckmessungen durchzuführen, und mit den Steuermitteln (6) zusammenwirken, um ihnen die Druckmessung(en) zu liefern.

8. Beatmungsgerät nach Anspruch 1, **dadurch gekennzeichnet, dass** die Steuermittel (6) mindestens einen Mikroprozessor (6.2) umfassen, wobei vorzugsweise der mindestens eine Mikroprozessor (6.2) auf einer Elektronikkarte (6.1) angeordnet ist.

9. Beatmungsgerät nach Anspruch 1, **dadurch gekennzeichnet, dass** er eine CO$_2$-Gehalt-Messvorrichtung (9) umfasst, um den CO$_2$-Gehalt im aus den Lungen des Patienten austretenden Gas zu messen und die CO$_2$ an die Steuermittel (6) zu übertragen, und die Steuermittel so konfiguriert sind, dass sie die CO$_2$-Werte verarbeiten und eine Anzeige einer CO$_2$-Kurve über die Zeit auf dem Anzeigebildschirm (8) steuern.

10. Beatmungsgerät nach Anspruch 1, **dadurch gekennzeichnet, dass** der Gaskreislauf (3) fluidisch mit einer Atemschnittstelle (10), vorzugsweise einer Atemmaske oder einem Trachealintubationsschlauch, verbunden ist.

11. Beatmungsgerät nach einem der Ansprüche 1 oder 2, **dadurch gekennzeichnet, dass** der erste und der zweite niedrige Druck (PB, PB2) so sind, dass: PB1=PB2.

12. Beatmungsgerät nach Anspruch 9, **dadurch gekennzeichnet, dass** die Vorrichtung zur Messung des CO$_2$-Gehalts (9) ein Kapnometer ist.

13. Beatmungsgerät nach einem der Ansprüche 1 oder 4, **dadurch gekennzeichnet, dass**:

- die erste Frequenz (F1) zwischen 8 und 12 Zyklen/min liegt und/oder
- die zweite Frequenz (F2) zwischen 15 und 20 Zyklen/min liegt.

14. Beatmungsgerät gemäß einem der Ansprüche 1 oder 2, **dadurch gekennzeichnet, dass** der zweite Beatmungsmodus (MV2) durch Werte für einen zweiten niedrigen Druck (PB2) und einen zweiten hohen Druck (PH2) gekennzeichnet ist, die einer Beatmung entsprechen, die nach Erkennung eines RACS des Patienten durchgeführt werden soll.

15. Beatmungsgerät gemäß den Ansprüchen 1, 9 und 14, **dadurch gekennzeichnet, dass**:

- der Bildschirm (8) so konfiguriert ist, dass er die $CO_2$-Konzentrationskurve im Zeitverlauf anzeigt, und
- die Auswahl des zweiten Beatmungsmodus (MV2) durch den Benutzer über die Auswahlmittel (5; 5.1, 5.2) erfolgt, nachdem ein RACS des Patienten auf der angezeigten $CO_2$-Konzentrationskurve im Zeitverlauf angezeigt wurde, die durch einen deutlichen Anstieg der aus den Lungen des Patienten P austretenden $CO_2$-Menge gekennzeichnet ist.

**Claims**

1. A medical ventilator (1) configured to provide respiratory assistance to a person in cardiac arrest comprising:

- a motorized blower (2) configured to supply respiratory gas,
- a gas circuit (3) comprising an inspiratory branch (3.1) configured to convey the gas flow supplied by the motorized blower (2),
- means (4) for storing ventilation modes (MV1, MV2) configured to store several ventilation modes comprising at least:

- a first ventilation mode (MV1) to be implemented during cardiac massage, **characterized by** first low pressure (PB1) and first high pressure (PH1) values, with PH1>PB1, and
- a second ventilation mode (MV2) to be implemented in the absence of cardiac massage, **characterized by** second low pressure (PB2) and second high pressure (PH2) values, with PH2>PB2,

- means (5; 5.1, 5.2) for selecting a ventilation mode (MV1, MV2) allowing a user to select at least the first (MV1) or second (MV2) stored ventilation mode, and
- control means (6) configured to control the motorized blower (2) in response to the user's selection of one of said ventilation modes (MV1, MV2), to supply gas at low and high pressures (PB1, PH1; PB2, PH2) corresponding to the selected ventilation mode (MV1, MV2),

**characterized in that** the second stored ventilation mode (MV2) is **characterized by** a second high pressure value (PH2), such that : PH2 < PH1

2. Ventilator according to claim 1, **characterized in that**:

- the first low pressure (PB1) is between 0 and 10 cmH$_2$O, preferably between 0 and 5 cmH$_2$O;
- the first high pressure (PH1) is between 10 and 40 cmH$_2$O, preferably between 20 and 30 cmH$_2$O;
- the second low pressure (PB2) is between 0 and 20 cmH$_2$O, preferably between 5 and 10 cmH$_2$O; and/or
- the second high pressure (PH2) is between 10 and 40 cmH$_2$O, preferably between 15 and 25 cmH$_2$O.

3. Ventilator according to claim 1, **characterized in that** the first ventilation mode (MV1) is further **characterized by** a first frequency (F1) and the second ventilation mode (MV2) is further **characterized by** a second frequency (F2), with F2>F1.

4. Ventilator according to claim 1, **characterized in that**:

- the first frequency (F1) is between 5 and 20 cycles/min and/or
- the second frequency (F2) is between 5 and 35 cycles/min.

5. Ventilator according to claim 1, **characterized in that** it comprises a digital display screen (8) displaying at least one virtual key (5.1, 5.2), the means for selecting (5; 5.1, 5.2) the ventilation mode comprising said at least one virtual key (5.1, 5.2).

6. Ventilator according to claim 1, **characterized in that** the ventilation mode storage means (4) (MV1, MV2) comprise at least one computer memory.

7. Ventilator according to claim 1, **characterized in that** it further comprises pressure measurement means (7) arranged in the gas circuit (3) to perform one or more pressure measurements therein and cooperating with the control means (6) to provide them with the pressure measurement(s).

8. Ventilator according to claim 1, **characterized in that** the control means (6) comprise at least one microprocessor (6.2), preferably said at least one microprocessor (6.2) being arranged on an electronic card (6.1).

9. Ventilator according to claim 1, **characterized in that** it comprises a $CO_2$ content measurement device

(9) for measuring the $CO_2$ content in the gas leaving the patient's lungs and transmitting the $CO_2$ measurements to the control means (6), and the control means are configured to process said $CO_2$ content measurements and control a display on the display screen (8) of a $CO_2$ content curve over time.

10. Ventilator according to claim 1, **characterized in that** the gas circuit (3) is fluidically connected to a respiratory interface (10), preferably a respiratory mask or a tracheal intubation tube.

11. Ventilator according to one of claims 1 or 2, **characterized in that** the first and second low pressures (PB, PB2) are such that: PB1=PB2.

12. Ventilator according to claim 9, **characterized in that** the $CO_2$ content measuring device (9) is a capnometer.

13. Ventilator according to one of claims 1 or 4, **characterized in that**:

   - the first frequency (F1) is between 8 and 12 cycles/min and/or
   - the second frequency (F2) is between 15 and 20 cycles/min.

14. Ventilator according to one of claims 1 or 2, **characterized in that** the second ventilation mode (MV2) is **characterized by** second low pressure (PB2) and second high pressure (PH2) values corresponding to ventilation to be implemented after detection of a RACS in the patient.

15. Ventilator according to claims 1, 9, and 14, **characterized in that**:

   - the display screen (8) is configured to display the $CO_-$ content curve over time, and

   - the selection of the second ventilation mode (MV2) by the user is performed via the selection means (5; 5.1, 5.2), after visualization of a RACS of the patient on the $CO_-$ content curve over time displayed, **characterized by** a notable increase in the amount of $CO_-$leaving the lungs of patient P.

EP 4 450 103 B1

## Figure 1

## Figure 2

13

Figure 3

Temps (min)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3636307 A
- EP 3218037 A

- EP 3323457 A